## Europäisches Patentamt
## European Patent Office
## Office européen des brevets

(19)

(11) Veröffentlichungsnummer: **0 138 175**
**B1**

(12) **EUROPÄISCHE PATENTSCHRIFT**

(45) Veröffentlichungstag der Patentschrift:
01.07.87

(51) Int. Cl.⁴: **C 07 H 3/04**

(21) Anmeldenummer: **84112008.2**

(22) Anmeldetag: **06.10.84**

(54) **Verfahren zur Kristallisation von Pentaacetylglukose.**

(30) Priorität: **14.10.83 DE 3337389**

(43) Veröffentlichungstag der Anmeldung:
**24.04.85 Patentblatt 85/17**

(45) Bekanntmachung des Hinweises auf die Patenterteilung:
**01.07.87 Patentblatt 87/27**

(84) Benannte Vertragsstaaten:
**DE FR GB IT**

(56) Entgegenhaltungen:
**US-A-2 857 378**

**CHEMICAL ABSTRACTS, Band 83, Nr. 1, 7. Juli 1975,
Seite 909, Nr. 10712m, Columbus, Ohio, US**

(73) Patentinhaber: **BASF Aktiengesellschaft, Carl-Bosch- Strasse 38, D-6700 Ludwigshafen (DE)**

(72) Erfinder: **Brunnmueller, Fritz, Dr., Alsenzstrasse 7,
D-6703 Limburgerhof (DE)**
Erfinder: **Schneider, Rolf, Dr., Feldbergstrasse 21,
D-6800 Mannheim 1 (DE)**
Erfinder: **Schuster, Werner, Maxburgstrasse 8,
D-6737 Boehl- Iggelheim (DE)**
Erfinder: **Vilhuber, Heinz Georg, Dr.,
Maulbeerstrasse 34, D-6720 Speyer (DE)**

## Beschreibung

Die Erfindung betrifft ein Kristallisationsverfahren für Pentaacetylglukose, bei dem man eine Lösung von Pentaacetylglukose in Essigsäure gleichzeitig mit Wasser in eine Maische aus kristalliner Pentaacetylglukose in wäßriger Essigsäure unter bestimmten Verhältnissen und Rühren einlaufen läßt und das in großtechnischem Maßstab diskontinuierlich und kontinuierlich ausgeführt werden kann.

Es ist bekannt, daß Ester von Polyalkoholen, wie z. B. Cellulose, Stärke und Zucker, hergestellt werden können, indem man den Polyalkohol mit Acetanhydrid in Gegenwart eines sauren oder basischen Katalysators, wie beispielsweise Schwefelsäure, Perchlorsäure, Zinkchlorid, Pyridin, Natriumhydroxid oder Natriumacetat, umsetzt. Neuerdings werden zur Beschleunigung der Reaktion auch wasserfreies Eisen-III-chlorid in Kombination mit anderen zwei- oder dreiwertigen Metallsalzen, wie z. B. Aluminium oder Zinkchlorid verwendet (GB 1 585 200, Carbohydrate Research 80 (1980) 346-349).

Die genannte Literatur befaßt sich praktisch ausschließlich mit der Synthese von acetylierten Polyalkoholen, d.h. mit der eigentlichen Acetylierung, und nicht mit der auch für die Praxis wichtigen Forderung, daß bei einer technischen Verwendung die Acylierungsprodukte in kristalliner Form vorliegen müssen. Kristallin werden die acetylierten Zucker üblicherweise durch Eingießen der Reaktionslösung in Eiswasser erhalten. In der Regel bilden sich die Kristalle nur sehr langsam. Anschließend wird abfiltriert. Diese Verfahrensweise dann für acetylierte Zucker eigentlich nur im Labormaßstab betrieben werden.

Weiterhin wird beispielsweise in der US-A-2 857 378 die Kristallisation von Pentaacetylglykose unter Rühren in 5- bis 50-gew.%iger wäßriger Essigsäure bei Temperaturen von 30° C und darüber, bevorzugt bei 45 bis 60° C, beschrieben. Die JP-A-74 133 314 lehrt, bei der Kristallisation konzentrierter Lösungen von Pentaacetylglukose Scherkräfte anzuwenden.

Pentaacetylglukose wird in bekannter Weise als Katalysator des Perboratzerfalles in Waschmitteln eingesetzt und ermöglicht als sogenannter Kaltbleichaktivator eine Bleichwirkung bereits bei 40 bis 60° C anstatt der bei 80 bis 90° C. Für den Einsatz in Waschpulvern werden hohe Anforderungen an die chemische und optische Reinheit der Pentaacetylglukose gestellt, z. B. soll das Produkt chemisch rein und gegebenenfalls gezielt einfärbbar sein.

Da es sich bei der Verwendung in Waschmitteln um große Mengen Pentaacetylglukose handelt, ist Aufgabe der vorliegenden Erfindung, ein möglichst rationelles großtechnisches Verfahren für die Kristallisation und Abtrennung von Pentaacetylglukose in hoher kristalliner Ausbeute, in hoher Raum/Zeit-Ausbeute und hoher Reinheit zu entwickeln. Dabei soll bei der kristallinen Pentaacetylglukose eine Korngrößenverteilung $\leqslant$ 300 µm erreicht werden, da mit dieser Teilchengröße bei der Anwendung als Kaltbleichaktivator eine optimale Wirkung erzielt wird.

Gegenstand der Erfindung ist ein Verfahren zur Kristallisation von Pentaacetylglukose in wässrigen Essigsäure unter Rühren, das dadurch gekennzeichnet ist, daß man eine Lösung von Pentaacetylglukose in Essigsäure, die 10 bis 50 Gew.% Essigsäure enthält und eine Temperatur von 30 bis 80° C aufweist, gleichzeitig mit Wasser in eine Maische aus 10 bis 30 Gew.% kristalliner Pentaacetylglukose in 10 bis 40 gew.%iger wäßriger Essigsäure von einer Temperatur von 10 bis 30° C unter Führen in einem solchen Verhältnis einlaufen läßt, daß während der Kristallisation ein Essigsäuregehalt von 10 bis 40 Gew.%, Temperaturen von 10 bis 30° C und ein Gehalt an kristalliner Pentaacetylglukose von 10 bis 30 Gew.% eingehalten werden und daß man anschließend die kristalline Pentaacetylglukose in an sich üblicher Weise isoliert.

In der Regel wird die kristalline Pentaacetylglukose im Anschluß an die Kristallisation nach einer mittleren Verweilzeit der Kristalle in der wäßrigen Essigsäure von 30 bis 90 Minuten isoliert.

Bei der Pentaacetylglukose in Essigsäure, von der ausgegangen wird, handelt es sich in der Regel um die Reaktionslösung, die sich aus der bekannten Umsetzung von Glukose mit Essigsäureanhydrid in Gegenwart von Schwefelsäure als Katalysator ergibt und die durch Abdestillieren von Essigsäure unter vermindertem Druck eingeengt wird. Für eine vorteilhafte Fällung unter den erfindungsgemäßen Bedingungen wird so weit eingeengt, daß die Essigsäure in der Reaktionslösung in einer Konzentration von 10 bis 50 Gew.%, insbesondere von 20 bis 40 Gew.%, vorliegt. Besonders gute Ergebnisse werden in dem bevorzugten Bereich von 28 bis 35 Gew.% erreicht. Es sei erwähnt, daß Restmengen von Essigsäureanhydrid bis zu 5 Gew.% nicht störend wirken. Die Reaktionslösung soll während des Zulaufens in die vorgelegte Maische eine Temperatur von 30 bis 80° C, insbesondere von 40 bis 60° C und besonders bevorzugt von 40 bis 50° C aufweisen.

Mit der essigsauren Lösung von Pentaacethylglukose wird gleichzeitig Wasser zu der vorlegten Maschine aus Kristalliner Pentaacetylglukose und wäßriger Essigsäure gegeben. Dabei regelt man den Zulauf von Reaktionslösung und Wasser in der Weise, daß in der Maschine stets eine wäßrige Essigsäure mit einem Essigsäuregehalt von 10 bis 40 Gew.%, vorteilhaft von 15 bis 30 Gew.% und besonders bevorzugt von 18 bis 22 Gew.% vorliegt.

Neben dem Zulauf-Verhältnis der Reaktionslösung von Pentaacetylglukose in Essigsäure und des Wassers zu der vorgelegten essigsauren wäßrigen Maische und neben der einzuhaltenden Essigsäurekonzentration ist für ein optimales Kristallisieren ein weiteres entscheidendes Merkmal die Menge kristalliner Pentaacetylglukose in der Maische, die im Bereich von 10 bis 30 Gew.% und bevorzugt im Bereich von 23 - 27 Gew.% liegt. Auch während der Kristallisation soll die Menge kristalliner Pentaacetylglukose in der Maische in den oben genannten Bereichen gehalten werden. Zusätzlich zu den genannten Kriterien sind die bei der Fällung einzuhaltenden Temperaturen von entscheidender Bedeutung. Sie sollen 10°C nicht unter- und 30°C nicht überschreiten und bevorzugt in einem Temperaturbereich von 15 bis 20°C liegen. Falls notwendig, kann die Temperatur durch äußere Kühlung oder Eiszugabe geregelt werden.

Nur unter diesen speziellen Bedingungen werden die günstigen Ergebnisse erreicht.

Gegebenenfalls kann während der Kristallisation beispielsweise durch zusätzliche Zugabe von verdünnter wäßriger Essigsäure oder durch Rückführung der Mutterlauge der Gehalt an kristalliner Pentaacetylglukose in dem Kristallisationskessel auf die bevorzugte Konzentration von 18 bis 22 Gew.% eingestellt werden.

Das erfindungsgemäße Verfahren kann kontinuierlich oder diskontinuierlich durchgeführt werden.

Das erfindungsgemäße Verfahren liefert unter den ganz speziellen Bedingungen in einfacher und wirtschaftlicher Weise kristalline, leicht filtrierbare Pentaacetylglukose in ausgezeichneter Ausbeute und Reinheit. Angesichts der erfindungsgemäßen Bedingungen ist es überraschend, daß die Fällung leicht und mit besten Ergebnissen erfolgt, da nach bekannten Verfahren keine rasche Kristallisation und eine schlechte Raum-Zeit-Ausbeute zu erwarten war. Gleichzeitig werden ohne zusätzliche Verfahrensmaßnahmen Korngrößenverteilungen $\leqslant$ 300 µm erhalten. Durch die erfindungsgemäßen Bedingungen ist gewährleistet, daß die Pentaacetylglukose auf den üblichen zur Verfügung stehenden Apparaturen, wie z. B. Nutsche, Filterpresse oder Bandfilter, schnell und wirtschaftlich von der Mutterlauge abgetrennt und von anhaftender Essigsäure sowie gegebenenfalls Spuren von Essigsäureanhydrid durch Waschen mit Wasser, gegebenenfalls unter Zusatz von Ammoniak oder anderen alkalischen Medien, befreit werden kann.

Zur Trocknung der erhaltenen Pentaacetylglukose können die üblichen Trocknungsapparate eingesetzt werden, soweit eine Trocknungstemperatur unterhalb des Schmelzpunkts eingehalten wird. Im Hinblick auf die Verwendung in Waschmitteln ist es ganz besonders vorteilhaft, wenn als Trocknungsapparat ein Spin-Flash eingesetzt wird. Aufgrund der prinzipiellen Charakteristik dieses Trocknungsapparates kann die Trocknung so gesteuert werden, daß ein Endprodukt anfällt,

dessen Korngrößenverteilung (KV $\leqslant$ 300 µm) ideal für den technischen Verwendungszweck ist und ein zusätzlicher Mahlvorgang in einer Mühle sich erübrigt. Dies ist von besonderem Vorteil bei kontinuierlicher Durchführung des Verfahrens.

Die erhaltene Pentaacetylglukose läßt sich mit Farbstoffpigmenten, falls erforderlich, außerordentlich günstig einfärben, wenn man eine flüssige Pigmentpräparation während der Fällung oder nach beendeter Fällung zugibt, Eine Einfärbung nach erfolgter Abtrennung der Kristallmaische von der Mutterlauge beispielsweise durch Aufsprühen einer flüssigen Pigmentpräparation oder die Zugabe von flüssigen oder pulverförmigen Pigmentpräparationen oder Pigmentpulver zu der getrockneten Ware führt interessanterweise nicht zu einem akzeptablen Ergebnis.

## Beispiel 1

5 995 kg Essigsäureanhydrid werden auf 45 bis 50°C aufgeheizt. Es werden unter Rühren 1,2 kg konz. Schwefelsäure und 200 kg Glukose zugegeben. Die Temperatur steigt auf 60°C. Unter Kühlung werden weitere 1 800 kg Glukose so zugegeben, daß die Temperatur 60°C nicht über- und 50°C nicht unterschreitet. Dabei werden zusätzlich 2,4 kg konz. Schwefelsäure als Katalysator zudosiert Es wird 2 Std. bei 60°C nachreagiert und anschließend werden bei 65°C 1500 kg Essigsäure bei 30 mm Hg abdestilliert. Die Reaktionslösung (31,2 Gew.% Essigsäuregehalt) wird auf 45°C abgekühlt.

Anschließend läßt man die Reaktionslösung mit einer Geschwindigkeit von 1 000 kg/h und Wasser mit 1 250 kg/h (Verhältnis 1 : 1,25) in eine vorgelegte Maische aus 1 500 l 20 %iger wäßriger Essigsäure und 300 kg kristalliner Pentaacetylglukose einlaufen. Die Temperatur der Maische wird durch Kühlung auf 15 bis 20°C gehalten. Es entsteht ein kristalliner Niederschlag, der nach beendetem Zulauf noch 1 h nachgerührt und anschließend über eine Kastennutsche abfiltriert wird (Dauer ca. 1 h). Es wird mit 12 m³ Wasser neutral gewaschen (Dauer: ca. 4 h) und in einem Vakuumsxchaufeltrockner bei 70 bis 80°C und 100 mm Hg in 18 Std. getrocknet.

Ausbeute: 3 685 kg = 85 % d. Theorie, bezogen auf eingesetzte Glukose.

Schmp. 92 bis 93°C farblose Kristalle Absorption bei 400 nm (1 cm, Kuvette 5 %ige Lösung in Dimethylformamid): 0,047 Reinheit HPLC 99,9 %,

Essigsäuregehalt: < 0,02 %.

Die gemessene Absorption und die Hochdruckgaschromatographie belegen die hervorragende Reinheit.

## Vergleichsbeispiele

I. Ansatz der Reaktionslösung wie in Beispiel 1. Es werden 2 350 kg Essigsäure abdestilliert und die Reaktionslösung (20,8 % Essigsäuregehalt) wird auf 45°C abgekühlt.

Anschließend läßt man 1 000 kg/h Reaktionslösung und 2 300 kg/h Wasser (Verhältnis 1: 2,3) in eine vorgelegte Maische aus 1 500 l 20%ige wäßrige Essigsäure und 200 kg kristalliner Pentaacetylglukose einlaufen. Die Temperatur der Maische wird durch Zugabe von Eis auf 18 bis 20°C gehalten.

Es entstehen grobe kugelförmige Agglomerate, die nach beendetem Zulauf noch 1 h nachgerührt werden und anschließend über eine Kastennutsche abfiltriert wird (Dauer ca. 1 Stunde).

Es wird mit etwa 60 m$^3$ Wasser neutral gewaschen (Dauer: ca. 22 h) und in einem Vakuumschaufeltrockner bei 60 bis 70°C und 100 mm Mg in 28 Std. getrocknet (starker Essigsäuregeruch).

Ausbeute: 3 380 kg = 78 % d. Theorie, bezogen auf eingesetzte Glukose.

Schmp: 89 bis 90°C, nicht ganz farblose Kristalle

Absorption bei 400 nm (1 cm Küvette 5 %ige Lösung in Dimethylformamid) 0,174

Reinheit HPLC; 97,5 %

Essigsäuregehalt: 0,2 %

Dieses Beispiel zeigt, daß schon bei geringen Änderungen der Konzentration der wäßrigen Essigsäure in der Maische grobkristalline Teilchen mit unbefriedigender Reinheit entstehen. Wasch- und Trockenzeiten verlängern sich drastisch, so daß eine befriedigende Raum/Zeit-Ausbeute nicht mehr vorhanden ist. Das Produkt riecht stets noch nach Essigsäure.

II. Reaktionsansatz wie Beispiel 1. Es werden 2300 l Essigsäure bei 30 mbar Hg abdestilliert. Die Reaktionslösung (20,9 % Essigsäuregehalt) wird auf 45°C abgekühlt.

Anschließend läßt man die 1 000 kg/h Reaktionslösung und 800 kg/h Wasser (Verhältnis 1: 0,8) in eine vorgelegte Maische aus 1 000 l 20 %iger wäßriger Essigsäure und 400 kg kristalliner Pentaacetylglukose einlaufen. Die Temperatur der Maische wird durch Zusatz von Eis auf 18 bis 20°C gehalten. Es entsteht ein feinstkristalliner, z. B. schleimiger Niederschlag, der nach beendetem Zulauf noch 2 Stunden nachgerührt wird und anschließend über eine Kastennutsche abfiltriert wird. Die Filtration gestaltet sich sehr schlecht und dauert 6 bis 8 Stunden. Die Waschzeit mit Wasser bis zum Neutralpunkt dauert ca. 60 bis 80 Stunden.

Der Niederschlag wird in einem Schaufeltrockner bei 70 bis 80°C und 100 mm Hg getrocknet. Dauer: 32 Stunden.

Ausbeute: 3 352 kg = 77,4 % d. Theorie, bezogen auf eingesetzte Glukose,

Schmelzpunkt 90 - 92°C, Absorption bei 400 nm (1 cm Küvette 5 %ige Lösung in DMF): 0,021, Essigsäuregehalt: 0,06 % (riecht nach Essigsäure).

Auch dieses Beispiel zeigt, daß bei geringen Änderungen des Gehaltes an kristalliner Pentaacetylglukose in der Maische keine befriedigende Raum/Zeit-Ausbeute erhalten wird und das Produkt stets mit Essigsäure verunreinigt ist.

III. 2 792 kg Essigsäureanhydrid werden auf 45 bis 50°C aufgeheizt. Es werden 0,67 kg Schwefelsäure und 138 kg Glukose zugegeben. Die Temperatur steigt auf 60°C. Unter Kühlung werden weitere 800 kg Glukose so zugegeben, daß die Temperatur 60°C nicht über- und 50°C nicht unterschreitet. Dabei werden zusätzlich 1 kg konz. Schwefelsäure als Katalysator zudosiert. Es wird 2 Stunden bei 60°C nachreagiert und anschließend auf 15°C abgekühlt. Nun werden 500 l Wasser innerhalb von 1 Stunde zugegeben. Es werden 30 Minuten nachgerührt und anschließend weitere 4 000 l Wasser portionsweise über einen Zeitraum von 3 Stunden zugegeben.

Der dicke Kristallbrei wird auf eine Kastennutsche abgelassen und mit viel Wasser neutral gewaschen (60 m$^3$ 20 Stunden). Der Niederschlag wird im Schaufeltrockner bei 70°C und 100 mm Hg getrocknet (Dauer: 31 Stunden).

Ausbeute: 1 500 kg = 73,8 % d. Theorie, bezogen auf eingesetzte Glukose. Absorption bei 400 nm (2 cm Küvette 5 %ige Lösung in DMF): 0,26 Reinheit im HPLC: 97,9 %, Schmp. 91 bis 93°C.

Dieses Beispiel zeigt, daß unter den Bedingungen der Kristallisation, wie sie für acetylierte Zucker in der Literatur im Labormaßstab beschrieben ist, keine befriedigende Raum/Zeit-Ausbeute, sowie Reinheit und Ausbeute zu erzielen ist.

## Patentansprüche

1. Verfahren zur Kristallisation von Pentaacetylglukose in wässrigen Essigsäure unter Rühren, dadurch gekennzeichnet, daß man eine Lösung von Pentaacetylglukose, die 10 bis 50 Gew.% Essigsäure enthält und eine Temperatur von 30 bis 80°C aufweist, gleichzeitig mit Wasser in eine Maische aus 10 bis 30 Gew.% kristalliner Pentaacetylglukose in 10 bis 40 gew.%iger wäßriger Essigsäure von einer Temperatur von 10 bis 30°C unter Rühren in einem solchen Verhältnis einlaufen läßt, daß während der Kristallisation ein Essigsäuregehalt von 10 bis 40 Gew.%, Temperaturen von 10 bis 30°C und ein Gehalt an kristalliner Pentaacetylglukose von 10 bis 30 Gew.% eingehalten werden und daß man anschließend die kristallisierte Pentaacetylglukose in an sich üblicher Weise isoliert.

2. Verfahren nach Anspruch 1, dadurch gekennzeichnet, daß bei der Kristallisation in der Maische ein Essigsäuregehalt von 18 bis 22 Gew.%, Temperaturen von 15 bis 20°C und ein Feststoffgehalt an Pentaacetylglukose von 23 bis 27 Gew.% eingehalten wird.

3. Verfahren nach Anspruch 1 oder 2, <u>dadurch gekennzeichnet</u>, daß als Lösung von Pentaacetylglukose in Essigsäure die Reaktionslösung der Umsetzung von Glukose mit Essigsäureanhydrid verwendet wird.

## Claims

1. A process for the crystallization of pentaacetyl glucose in aqueous acetic acid, with stirring, wherein a solution of pentaacetyl glucose containing from 10 to 50 % by weight of acetic acid and having a temperature of from 30 to 80°C is allowed to run, together with water, into a suspension of from 10 to 30 % by weight of crystalline pentaacetyl glucose in 10 to 40 % strength by weight aqueous acetic acid at a temperature of from 10 to 30°C, with stirring, in such a ratio that an acetic acid content of from 10 to 40 % by weight, a temperature of from 10 to 30°C and a content of crystalline pentaacetyl glucose of from 10 to 30 % by weight are maintained, and wherein the crystallized pentaacetyl glucose is subsequently isolated in a conventional manner.

2. A process as claimed in claim 1, wherein an acetic acid content of from 18 to 22 % by weight, a temperature of from 15 to 20°C and a solids content of pentaacetyl glucose of from 23 to 27 % by weight are maintained during the crystallization in the suspension.

3. A process as claimed in claim 1 or 2, wherein the solution from the reaction of glucose with acetic anhydride is used as the solution of pentaacetyl glucose in acetic acid.

## Revendications

1. Procédé de cristallisation de pentaacétylglucose, sous agitation, dans de l'acide acétique aqueux, caractérisé par le fait que l'on fait couler, sous agitation, une solution de pentaacétylglucose, qui contient 10 à 50 % en poids d'acide acétique et possède une température de 30 à 80°C, en même temps que de l'eau, dans une masse de mélange de 10 à 30 % en poids de pentaacétylglucose cristallin dans de l'acide acétique à 10 à 40 % en poids, d'une température de 10 à 30°C, en rapport tel que, pendant la cristallisation, soient maintenues une teneur en acide acétique de 10 à 40 % en poids, des températures de 10 à 30°C et une teneur en pentaacétylglucose cristallin de 10 à 30 % en poids et on isole ensuite le pentaacétylglucose cristallisé, de manière habituelle en soi.

2. Procédé selon la revendication 1, caractérisé par le fait que, lors de la cristallisation dans la masse de mélange, sont maintenues une teneur en acide acétique de 18 à 22 % en poids, des températures de 15 à 20°C et une teneur en solides de pentaacétylglucose de 23 à 27 % en poids.

3. Procédé selon la revendication 1 ou 2, caractérisé par le fait que l'on utilise, comme solution de pentaacétylglucose dans l'acide acétique, la solution de réaction du glucose avec l'anhydride acétique.